# EUROPEAN PATENT SPECIFICATION

(11) **EP 3 990 083 B1**
(45) Date of publication and mention of the grant of the patent: **29.03.2023**
(21) Application number: 20734077.9
(22) Date of filing: 26.06.2020
(51) Int. Cl.: A61M 25/00, A61M 25/01, A61M 25/06, A61B 17/221, A61B 17/34

(54) **A DELIVERY CATHETER DEVICE AND A SYSTEM FOR ACCESSING THE INTRACRANIAL VASCULATURE**
ABGABEKATHETERVORRICHTUNG UND SYSTEM ZUM ZUGRIFF AUF DAS INTRAKRANIELLE GEFÄSSSYSTEM
DISPOSITIF DE CATHÉTER DE DÉLIVRANCE ET SYSTÈME D'ACCÈS À LA VASCULATURE INTRACRÂNIENNE

(30) Priority: 28.06.2019 EP 19382551
(43) Date of publication of application: 04.05.2022
(73) Proprietor: Anaconda Biomed, S.L., 08174 Sant Cugat del Vallès (ES)
(72) Inventor: RÍOS GARRIGA, Anna, 08174 Sant Cugat del Vallès (ES); LIZARAZU GONZALEZ, Ane, 08013 Barcelona (ES); ARAD HADAR, Ofir, 08197 Sant Cugat del Vallès (ES); GALVE MURILLO, Iñaki, 08012 Barcelona (ES)
(74) Representative: Segui Quetglas, Margalida
(86) International application number: PCT/EP2020/068097
(87) International publication number: WO 2020/260632

(56) References cited:
- US-A1- 2004 243 102
- US-A1- 2015 164 666
- US-A1- 2015 231 360
- US-A1- 2018 193 042

## Description

### Technical Field

The present invention is directed, in general, to the field of medical systems. In particular, the invention relates to a delivery catheter device, and to a system, for accessing the intracranial vasculature to enable the introduction and expansion of a movable medical device therein.

### Background of the Invention

Delivery catheters are used in a variety of medical procedures to provide access for the purpose of stent, guidewire, catheter, and fluid delivery and placement.

There are known some patent and patent applications in this field.

WO 2009014723-A1 provides a delivery catheter for accessing the intracranial vasculature that includes a rigid proximal section and a distal section having an outer diameter and flexibility suitable for advancement into the intra-cranial vasculature, such as the petrous segment or the cavernous segment of the internal carotid artery. The wall thickness and rigidity of the catheter decrease from the proximal section to the distal section, preferably in discrete segments, where each has reduced wall thickness and/or durometer relative to proximally adjacent sections. An intra-cranial access system includes the delivery catheter and a selection catheter insertable through the lumen of the delivery catheter. The selection catheter is shaped to facilitate the selection of the target branch of the neurovasculature off the aortic arch and allows the delivery catheter to be advanced over the selection catheter into the selected branch.

US 2018361114-A1 discloses a rapid exchange microcatheter for accessing the intracranial neurovasculature. A side opening through the sidewall is located a first distance proximal to the distal-most tip. A proximal opening located proximal to the side opening is a second distance from the distal-most tip. A distal, reinforced catheter portion extends between a distal end region to a point near the side opening. A proximal, reinforced catheter portion extends a distance from a point near the side opening towards the proximal end of the catheter body. The side opening is positioned within a gap between a proximal end of the distal reinforced catheter portion and a distal end of the proximal reinforced catheter portion. A payload of a cerebral treatment device is housed within the lumen proximal to the side opening.

US 6752819-B1 discloses a medical catheter for the transvascular deployment of a collapsible medical device such as a filter having a tubular body formed by an inner tubular core surrounded by an outer thin-walled tube which is fixed to the core. The outer thin-walled tube extends outwardly beyond a distal end of the core to form a fixed thin-walled medical device embracing pod. The filter is carried on a guidewire which is slidably engageable within a central lumen of the core and the filter can be collapsed against the guidewire for loading within the pod. With the filter thus loaded within the pod the distal end of the catheter can be maneuvered through a patient's vascular system to a desired deployment site where the filter is discharged from the pod allowing the filter to expand within the blood vessel for use filtering blood flowing through the blood vessel.

WO 2018160966-A1 relates to catheter devices having different structural property regions and to related systems and methods. The catheter devices can be implemented in systems and methods for accessing cerebral cisterns and draining cerebrospinal fluid (CSF), (e.g., to relieve elevated intracranial pressure), using an endovascular approach. More on particular, the catheter devices can include: a reinforcing member having a proximal and distal ends, the reinforcing member comprising: discrete longitudinally arranged structural regions between the proximal and distal ends comprising: a first, proximal, structural region defining a first series of wall perforations that generate structural properties within the first structural region, the first series of wall perforations setting a first stiffness of the first structural region; and a second structural region, disposed distally relative to the first structural region, defining a second series of wall perforations that generate structural properties within the second structural region, the second series of wall perforations setting a second stiffness of the second structural region, which is less than the first stiffness, wherein the second series of wall perforations differs from the first series of wall perforations by at least one of: cut balance, cut frequency, or pitch.

WO 2013152327-A1 provides a system and method for treating an aneurysm or other vessel disease or defect. The system includes an expandable device for placement in a vessel, where the mechanically expandable device includes a membrane. Also disclosed is a delivery device constructed and arranged to position the expandable device such that the exterior surface of the expandable device engages with the inner surface of the vessel and maintains a fluid pathway through said vessel.

US 2004243102-A1 discloses a guiding catheter for use in coronary angioplasty and other cardiovascular interventions which incorporates a plurality of segments of selected flexural modulus in the shaft of the device. The segments have a different flexibility than the sections immediately proximal and distal to them, creating zones in the catheter shaft which are either more or less flexible than other zones of the shaft. The flexibility and length of the shaft in a given zone are then matched to its clinical function and role. A mid-shaft zone is significantly softer than a proximal shaft or distal secondary curve to better traverse the aortic arch shape without storing too much energy. A secondary zone section is designed to have maximum stiffness to provide optimum backup support and stability. The relation between the length of the different segments and their corresponding durometer of this guiding catheter makes it practical for coronary angioplasty and other cardiovascular interventions. However, this relation is unfeasible for navigating the tortuous anatomy of the brain.

US 2015164666-A1 relates to an intravascular intervention system that includes a manipulation member sized for insertion into a blood vessel. The manipulation member can include a longitudinally extending tube having a helical cut extending along the tube. The helical cut can have an axial length of at least 50 cm and be continuous along the axial length. The intervention member can be compressible to a collapsed configuration for delivery to an endovascular treatment site through a catheter and be self-expandable from the collapsed configuration to an expanded configuration.

US 2015231360-A1 discloses a shaft of a catheter. The shaft has a thin portion, which constitutes a distal side of the shaft, a thick portion which is provided further on a proximal side than the thin portion, and a tapered portion which is provided between the thin portion and the thick portion. The tapered portion is formed more flexibly than the thick portion. The thick portion has a first thick portion and a second thick portion which is provided between the tapered portion and the first thick portion. The second thick portion has a smaller outer diameter than the outer diameter of a proximal end of the tapered portion and the outer diameter of the first thick portion and is formed more flexibly than the first thick portion.

US 2018193042-A1 describes methods, systems, and devices for facilitation of intraluminal medical procedures within the neurovasculature. A catheter advancement device includes a flexible elongate body having a proximal portion coupled to a proximal end region of the flexible elongate body and extending proximally to a proximal-most end of the catheter advancement element. A hardness of the flexible elongate body transitions proximally towards increasingly harder materials up to the proximal portion forming a first plurality of material transitions. At least a portion of the flexible elongate body is formed of a plurality of layers including a reinforcement layer. An outer diameter of the flexible elongate body is sized to be positioned coaxially within a lumen of a catheter such that a distal tip portion of the flexible elongate body extends distally beyond a distal end of the catheter to aid in delivery of the catheter to an intracranial vessel.

Other delivery catheters are also known by US 2018126132-A1, WO 2017075544-A1 and US 9861783-B2.

### Description of the Invention

Delivery catheters for treating locations within the intracranial vasculature need to navigate within the tortuous anatomy of the brain and easily reach the different areas that need to be treated. Besides, these delivery catheters should be suitable to deliver and navigate a movable medical device beyond the carotid syphon of the brain without elongation of the delivery catheters.

To that end, one aspect of the invention provides a delivery catheter device for accessing the intracranial vasculature and for delivering a movable medical device beyond a carotid syphon. The delivery catheter has an elongated tubular body having a lumen, a proximal section, a distal section and at least three longitudinally arranged transition sections disposed between the proximal section and the distal section. The distal section is the most flexible section of the elongated tubular body, and the proximal section is the stiffest section of the elongated tubular body. A first transition section of the longitudinally arranged transition sections has a stiffness less than a stiffness of the proximal section, a second transition section of the longitudinally arranged transition sections is disposed distal to the first transition section and has a stiffness greater than the stiffness of the first transition section, and a third transition section of the longitudinally arranged transition sections is disposed distal to the second transition section and has a stiffness less than the stiffness of the second transition section. Likewise, the proximal section and the transition sections of the delivery catheter are adapted/configured to enable the distal section to be advanced beyond the carotid syphon by applying a distally directed force on the proximal section.

In some embodiments, the longitudinally arranged transition sections further comprise: a fourth transition section disposed distal to the third transition section and having a stiffness less than the stiffness of the third transition section and greater than the stiffness of the distal section, and a fifth transition section disposed proximal to the first transition section and having a stiffness less than, or equal to, the stiffness of the proximal section and greater than, or equal to, the stiffness of the first transition section.

The configuration of lengths and stiffness of each section allows the proposed delivery catheter device to be used for neurovascular access to navigate within the brain. In particular, the design/configuration (i.e., specific relation between length, stiffness and/or hardness) of the distal section and the third and second transition sections (or fourth, third and second sections depending on the specific embodiment) allows the delivery catheter device to take the shape of the tortuous anatomy of the brain and to easily reach the different areas that need to be treated.

Particularly, the proximal section and the transition sections of the delivery catheter are adapted/configured to enable the distal section to be advanced beyond the carotid syphon by applying a distally directed force on the proximal section when the movable medical device is disposed within the distal section.

According to the invention, the stiffness of the first transition section is less than the stiffness of the proximal section, the stiffness of the second transition section is greater than the stiffness of the first transition section, and the stiffness of the third transition section is less than the stiffness of the second transition section.

The term stiffness in this description is understood as commonly in the art as the rigidity of an object, the extent to which it resists deformation in response to an applied force. The stiffness of an object depends on different features, e.g. number of layers, type of materials, the presence or not of a reinforcing element, among others. The term flexibility is the opposite word of stiffness, i.e., a higher stiffness is equivalent to a lower flexibility.

The term durometer in this description is understood as commonly in the art as the value of hardness (i.e. shore D hardness) of an object, as a measure of how resistant solid matter is to various kinds of permanent shape change when a compressive force is applied on the surface of the object. The hardness of a material describes the surface property but can affect the stiffness of objects made from that material.

The elongated tubular body can have a length in a range between 900 - 1500 mm. In a particular embodiment, the elongated tubular body has a length of 1320 or 1350 mm.

In an embodiment, the distal section has a length of at least 80 mm.

In an embodiment, the sum of the lengths of the distal section and of the third transition is at least 120 mm.

In an embodiment, the distal section has a durometer up to 35D. In other embodiments, the distal section and the third transition section have a durometer up to 55D.

In some embodiments, the proximal section can have a durometer comprised in a range between 70-74D; the distal section can have a durometer in a range between 25-30D; the third transition section can have a durometer in a range between 40-60D; the second transition section can have a durometer in a range between 55-74D, particularly 60-72D; and the first transition section can have a durometer in a range between 55-74D, particularly 60-72D.

In another embodiment, the proximal section has a durometer of 74D, the distal section has a durometer of 29D, and the five transition sections have durometers of 72D, 55D, 72D, 55D, and 40D, respectively, starting from the transition section adjacent to the proximal section. In another embodiment, the proximal section has a durometer of 70D, the distal section has a durometer of 29D and the five transition sections have durometers of 72D, 55D, 72D, 55D, and 40D, respectively, starting from the transition section adjacent to the proximal section. In another embodiment, the proximal section has a durometer of 72 or 74D, the distal section has a durometer of 29D, and the five transition sections have a durometer of 72D, 70D, 72D, 55D and 40D, respectively, starting from the transition section adjacent to the proximal section. In another embodiment, the proximal section has a durometer of 70D, the distal section has a durometer of 29D, and the five transition sections have a durometer of 70D, 70D, 72D, 55D and 40D, respectively, starting from the transition section adjacent to the proximal section.

In some or all of these embodiments, the proximal section, the distal section and the at least three longitudinally arranged transition sections can be each made of a different material. For example, the proximal section can be made of a polyamide 12 material, the longitudinally arranged transition sections can be made of a polyether block amide material, and the distal section can be made of a thermoplastic polyurethane material.

In any of these embodiments, the elongated tubular body can have a diameter on the order of 4 to 6 French (the non-SI unit French used within this description is to be converted as follows: 1 French=1 /3 mm). The elongated tubular body can have an outer diameter in a range between 0.078" and 0.085" (i.e. 2.0-2.18 mm), for example 0.083" (i.e. 2.11 mm) and an inner diameter in a range between 0.064" - 0.073" (i.e. 1.65-1.85 mm), for example 0.072" (i.e. 1.82 mm). It should be noted that in other embodiments, the delivery catheter can have different diameters. The elongated tubular body can have a thickness of 0.011" (i.e. 0.28 mm).

The elongated tubular body can also include a hydrophilic coating, for example, Polyvinylpyrrolidone, Polyacrylamide, or combinations thereof, among others.

In any of these embodiments, the elongated tubular body can have different longitudinal layers. In an embodiment, the elongated tubular body comprises two layers, an inner layer, and an outer layer. In another embodiment, the elongated tubular body comprises three layers, an inner layer, an outer layer and an intermediate layer between the inner layer and the outer layer. The introduction of the intermediate layer allows a better fusion between the inner and outer layers.

The inner, outer, and intermediate layers of the elongated tubular body can be made of different materials. For example, the inner layer can be made of a polytetrafluoroethylene (PTFE) material; the outer and intermediate layers can be made of a polyamide material (e.g., Grilamid^{®}), a polyether block amide material (e.g. PEBAX^{®}) or a thermoplastic polyurethane material (e.g., Pellethane).

In any of these embodiments, the elongated tubular body can further include a reinforcing coil, wherein a section of the reinforcing coil in the transition sections has a stiffness that is different than a stiffness of a section of the reinforcing coil in the proximal section and a stiffness of a section of the reinforcing coil in the distal section.

In an embodiment, the reinforcing coil is disposed between the outer layer and the inner layer. In another embodiment, the reinforcing coil is disposed between the outer layer and the intermediate layer.

In any of these embodiments, the reinforcing coil, which is designed as a stainless-steel flat wire, can have a different coil pitch along the shaft of the overall length of the delivery catheter. In particular, the reinforcing coil pitch increases from the proximal section to the distal section, i.e., the distal section has a coil pitch higher (or open pitch) than the coil pitch of the proximal section (i.e. tight pitch at the proximal section). In a particular embodiment, the reinforcing coil comprises a 0.010" (0.254 mm) pitch at the proximal end, a 0.012" (0.3048 mm) pitch at the middle transition section and a 0.015" (0.381 mm) pitch at the distal end. In some embodiments, the coil pitch is maintained constant at the point between sections where the durometer changes, in order to avoid breakage of the elongated tubular body.

In some embodiments, the elongated tubular body also comprises a soft-tip zone disposed at the most distal end of the distal section. The reinforcing coil can be disposed over at least a portion of the soft-tip zone. Alternatively, the reinforcing coil can terminate before reaching the soft-tip zone (i.e., the soft-tip zone is not provided with the reinforcing coil). In some embodiments, the soft-tip zone can have a length between 0.5 and 5 mm, particularly of 2 mm, and a durometer of 25-30D, particularly 29D.

In any of these embodiments, the delivery catheter device can be adapted to hold the movable medical device within the elongated tubular body to place said movable medical device in an intracranial blood vessel to remove a thrombus, wherein the movable medical device is adapted to change its shape from a collapsed position within the elongated tubular body to an extended and expanded position at an opening of the distal section to remove said thrombus, such that the local stiffness of a portion of the delivery catheter device is altered by the movable medical device as it moves through that portion of the elongated tubular body. The interaction between the delivery catheter and the movable medical device allows the delivery catheter device to take the shape of the tortuous anatomy of the intracranial blood vessel and to easily reach the different areas that need to be treated.

In any of these embodiments, the delivery catheter device can also have an optional radiopaque marker at the distal section of the elongated tubular body to allow viewing a position of the delivery catheter device within the intracranial blood vessel under fluoroscopy.

Another aspect of the invention provides a system for accessing the intracranial vasculature and for delivering a movable medical device beyond a carotid syphon. The system includes a movable medical device and a delivery catheter device comprising an elongated tubular body having a lumen, a proximal section, a distal section and at least three longitudinally arranged transition sections disposed between the proximal section and the distal section; the distal section being the most flexible section of the elongated tubular body, the proximal section being the stiffest section of the elongated tubular body, and the at least three longitudinally arranged transition sections having a wall thickness having a durometer that varies between a durometer of the proximal section and of the distal section. The movable medical device is adapted/configured to change its shape from a collapsed position within the elongated tubular body to an extended and expanded position at an opening of the distal section to remove the thrombus. A first transition section of the longitudinally arranged transition sections has a stiffness less than a stiffness of the proximal section, a second transition section of the longitudinally arranged transition sections is disposed distal to the first transition section and has a stiffness greater than the stiffness of the first transition section, and a third transition section of the longitudinally arranged transition sections is disposed distal to the second transition section and has a stiffness less than the stiffness of the second transition section.

According to the invention, the stiffness of the first transition section is less than the stiffness of the proximal section, the stiffness of the second transition section is greater or equal than the stiffness of the first transition section, and the stiffness of the third transition section is less than the stiffness of the second transition section.

In some embodiments, the longitudinally arranged transition sections further comprise a fourth transition section disposed distal to the third transition and having a stiffness less than the stiffness of the third transition section and greater than the stiffness of the distal section, and a fifth transition section disposed proximal to the first transition section and having a stiffness less than, or equal to, the stiffness of the proximal section and greater than, or equal to, the stiffness of the first transition section.

Particularly, the stiffness of the fifth transition section is less than the stiffness of the proximal section and greater than the stiffness of the first transition section.

In an embodiment, the length of the distal section is at least 80 mm.

In an embodiment, the length of the distal section plus the length of the third transition section is at least 120 mm.

In an embodiment, the distal section has a durometer up to 35D.

In an embodiment, the distal section and the third transition section have a durometer up to 55D.

In some embodiments, the proximal section, the distal section and the at least three longitudinally arranged transition sections are each made of a different material. For example, the proximal section can be made of a polyamide 12 material, the longitudinally arranged transition sections can be made of a polyether block amide material and the distal section can be made of a thermoplastic polyurethane material.

In some embodiments, the elongated tubular body further comprises a reinforcing coil, wherein a section of the reinforcing coil in the transition sections has a stiffness that is different than a stiffness of a section of the reinforcing coil in the proximal section and a stiffness of a section of the reinforcing coil in the distal section.

In some embodiments, the movable medical device is configured to alter the stiffness of the delivery catheter device as it moves through the elongated tubular body. For example, in some embodiments the movable medical device can include a segment formed by a mesh of at least two sets of helicoidal filaments turning respectively in opposite directions and being intertwined, wherein the mesh comprises two distinct tubular sections, a first section and a second section, wherein the second section, adjacent to the first section provides a reduction of diameter, and wherein said mesh of the first section has a variable density of the helicoidal filaments configured to provide radial forces higher than in the second section, such that the first section becomes appositioned against an inner wall of the intracranial blood vessel. Optionally, the radial forces in first and second end portions of the first section can be higher than in an intermediate portion thereof; or, alternatively, the radial forces in the first section can be uniformly distributed along its generatrix.

In any of the system embodiments, the delivery catheter device and/or the mesh of the movable medical device can also have an optional radiopaque marker to allow viewing a position of the delivery catheter device within the intracranial blood vessel under fluoroscopy and/or to allow viewing when a distal section of the movable medical device is aligned with the distal section of the elongated tubular body.

Described herein, however not claimed, is a method of placing a distal section of a delivery catheter at a target location in the vasculature of a patient. In some examples, the method includes the steps of inserting the distal section through an insertion opening into the vasculature; advancing the distal section through the vasculature toward a tortuous section of a vessel in the vasculature; placing a transition section of the delivery catheter at a location just proximal to the tortuous section as the distal end enters the tortuous section, the transition section having a stiffness greater than a stiffness of an adjacent proximal section of the delivery catheter and an adjacent distal section of the delivery catheter; and advancing the distal end to the target location.

In some examples, the transition section is a second transition section, the delivery catheter further comprising a first transition section disposed proximal to the second transition section having a stiffness less or equal than the stiffness of the second transition section and a third transition section disposed distal to the second transition section and having a stiffness less than the stiffness of the second transition section.

In another example, the transition section of the delivery catheter further comprises the first transition section disposed proximal to the second transition section having a stiffness less than the stiffness of the second transition section and the third transition section disposed distal to the second transition section and having a stiffness less than the stiffness of the second transition section.

In some examples, a movable medical device is disposed in the delivery catheter, and the non-claimed method further includes the steps of disposing the movable medical device in the distal section of the delivery catheter while advancing the distal section through a first portion of the vasculature toward the target location, moving the movable medical device proximally in the delivery catheter to decrease the stiffness of the distal section of the delivery catheter, after the moving step, and advancing the distal section of the delivery catheter further toward the target location. The non-claimed method can also include, after moving the movable medical device proximally and advancing the distal section of the delivery catheter further distally, an optional step of moving the movable medical device distally in the delivery catheter to increase the stiffness of the distal section of the delivery catheter. In examples, the movable medical device is a self-expanding device having a collapsed delivery configuration and an expanded deployed configuration, and the movable medical device can apply a radially outward force on the delivery catheter when disposed in the delivery catheter in the delivery configuration.

In some examples, the target location is within an intracranial blood vessel, and the placing step includes the step of placing the transition section just proximal to a carotid syphon when the distal section is in the carotid syphon. In other embodiments, the target location is a M1 segment just distal to the carotid syphon, and the placing step includes the step of placing the transition section just proximal to a petrous or cavernous section when the distal section is disposed distal to the carotid syphon. In other examples, the target location is a basilar artery, and the placing step includes the step of placing the transition section just proximal to the basilar artery when the distal section is in the basilar artery.

In some such examples in which the movable medical device comprises a self-expanding device, the non-claimed method further includes the step of advancing the self-expanding device out of the delivery catheter, allowing the self-expanding device to self-expand, and capturing a thrombus in the self-expanding device.

According to the invention, the specific construction of the transition sections allows the delivery catheter to overcome different tortuosities in the brain vessels.

The above features concerning the second transition section enhance longitudinal force transmission from the proximal section to the distal section and avoid tension accumulation. In this way, all the force applied at the proximal section is transferred into movement at the distal section.

The foregoing and other objects, features and advantages of the invention will become more readily apparent from the following detailed description of embodiments of the invention, which proceeds with reference to the accompanying drawings, which must be considered in an illustrative and non-limiting manner.

### Brief Description of the Drawings

Fig. 1 is a view of a delivery catheter according to an embodiment of the present invention.
Fig. 2 is a view of a delivery catheter according to another embodiment of the present invention.
Fig. 3 illustrates a movable medical device to be hold within a delivery catheter according to embodiments of the present invention.
Fig. 4A illustrates an in-vitro 3D model showing the neurovascular anatomy.
Fig. 4B illustrates the neurovascular anatomy and the length ranges of each segment.
Fig. 5 illustrates the set-up scheme of the 3-point bending experiment done in Example 2.
Fig. 6A illustrates the flexibility profile (i.e. Peak Force [N] performed in 19 points along the catheter's length taking the distal end as the reference point) obtained for different catheters: ANA Delivery Catheter from Anaconda Biomed (ANA DC alone), ACE 68 from Penumbra and Sofia Plus from Terumo.
Fig. 6B illustrates the flexibility profile (i.e. Peak Force [N] performed in 19 points along the catheter's length taking the distal end as reference point) obtained for the ANA system in three different configurations: ANA Delivery Catheter alone (ANA DC alone), ANA Delivery Catheter in combination with the Funnel Catheter deployed (ANA DC + FC deployed), and ANA Delivery Catheter in combination with the Funnel Catheter with its distal tip at 5mm of the distal tip of the Delivery Catheter (ANA DC + FC 5 mm from tip).

### Detailed Description of the Invention and of Particular Embodiments

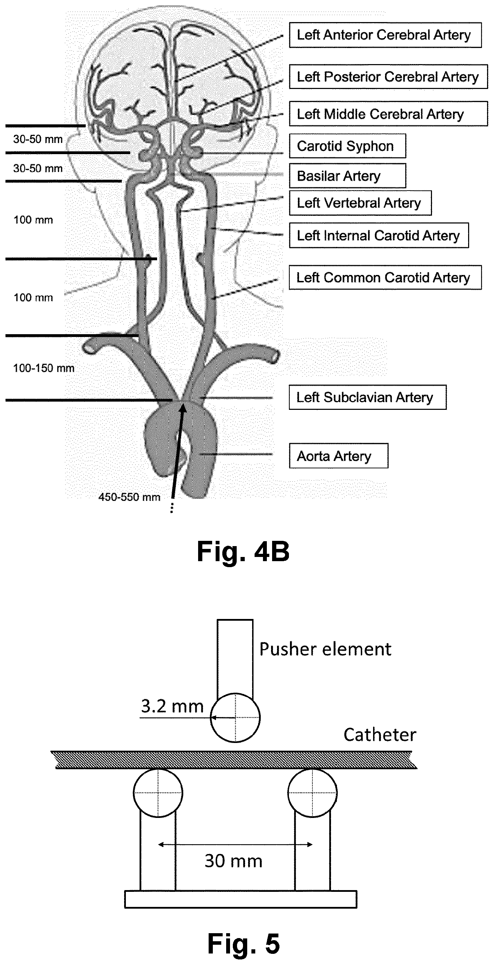

The delivery catheter of the invention is configured to reach a brain target location within an intracranial blood vessel. According to Fig. 4B, the delivery catheter is configured to be advanced beyond the carotid syphon reaching a cerebral artery (e.g. middle posterior or anterior cerebral artery). In an embodiment, the brain target location is a cerebral artery (e.g. located at least 30-50 mm distal to the carotid syphon), the distal section is disposed beyond the carotid syphon (e.g. located 30-50 mm distal to the basilar artery) and the transition section is disposed beyond the basilar artery (e.g at least 50-100 mm distal to the proximal-middle portion of the carotid artery).

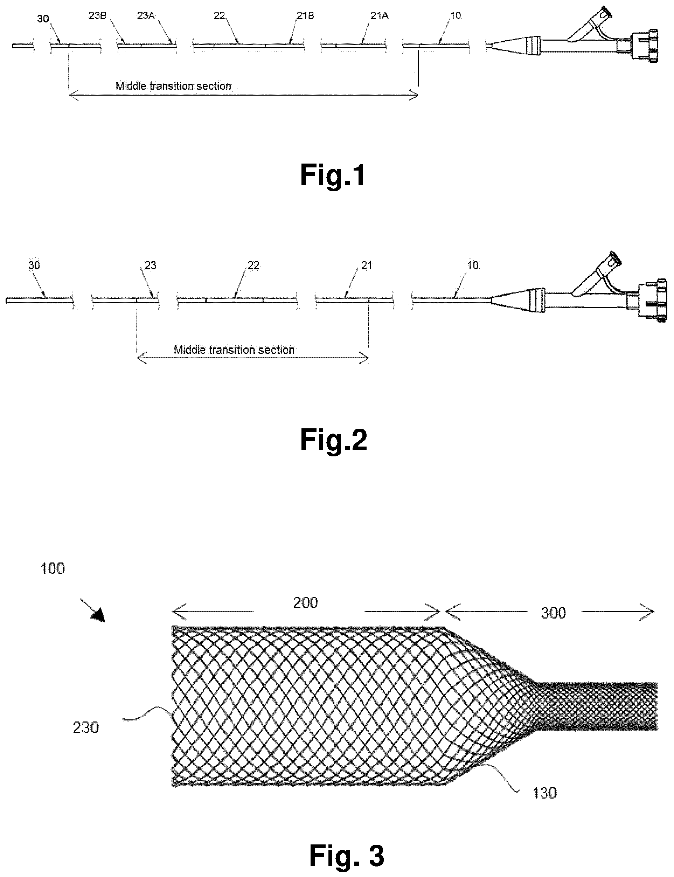

Referring to Figs. 1 and 2, these figures show different embodiments of the proposed delivery catheter device for accessing the intracranial vasculature. The delivery catheter device illustrated in Figs. 1 and 2 has an elongated tubular body having a lumen, a proximal section 10, a distal section 30 and a set of longitudinally arranged transition sections 20 disposed between the proximal section 10 and the distal section 30.

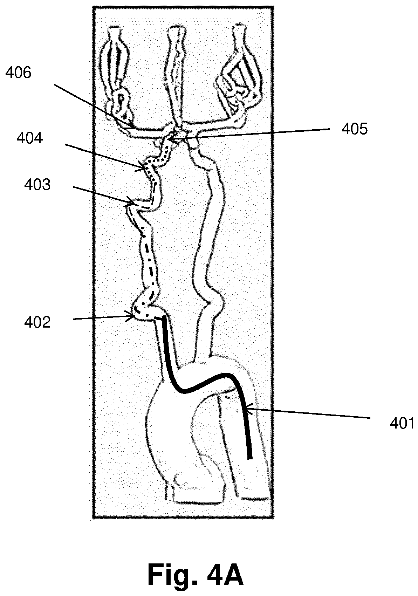

In either of the previous embodiments, the proximal section 10 is the stiffest section of the elongated body for robust pushability of the delivery catheter, and the distal section 30 is the most flexible section of the elongated tubular body to overcome tortuous paths in the vessels (i.e. to enhance navigation of the delivery catheter). The longitudinally arranged transition sections 20 include a first transition section that is less stiff than the proximal section 10, a second transition section distal to the first transition section that is more stiff than the first transition section, and a third transition section distal to the second transition section that is less stiff than the second transition section. The transition sections 20 are designed to enhance the ability of the delivery catheter to advance through tortuosities in the vessels. The longitudinal position and the length of each transition section are chosen to address the characteristics of the vasculature through which the delivery catheter will be advanced. For example, the third transition section can be placed at a longitudinal location that will be just proximal to a tortuous section of the vasculature (e.g., the carotid syphon 405, see Fig. 4A) to provide pushability as the flexible distal section 30 of the catheter passes through the tortuous vasculature, for example to reach the M1 segment 406 of the brain anatomy.

In some embodiments, the transition sections 20 have different durometers than the proximal and distal sections (e.g., by employing different materials) to avoid kinking/break points. For example, the proximal section 10 can be made of a polyamide 12 material such as Grilamid^{®} thermoplastic, the longitudinally arranged transition sections 20 can be made of a polyether block amide material (e.g. PEBAX^{®}) and the distal section 30 can be made of a thermoplastic polyurethane material such as Pellethane 80A. The durometer of the Grilamid material can range from 66 to 77 depending on its specific composition/type. In some embodiments, Grilamid L25 or L25H is used. In other embodiments, Grilamid TR90 or TR55 is used. In yet other embodiments, Grilamid LV is used. By using different materials for the different transition sections 20, each of the transition sections 20 can have a different stiffness without significantly altering the thickness of the catheter wall between adjacent sections.

The number and flexibility characteristics of transition sections 20 can be chosen to meet the clinical need. In the embodiment of Fig. 1, for example, the delivery catheter comprises five longitudinally arranged transition sections, referred in the figure as 21A, 21B, 22, 23A, and 23B. In this embodiment, the relative stiffness of each of the transition sections is affected by the hardness of the materials used in the transition sections. Thus, the middle transition section 22 has a higher durometer than its adjacent, previous 21B and next 23A, transition sections. In the embodiment of Fig. 2, the delivery catheter comprises three longitudinally arranged transition sections, referred to 21, 22 and 23. The middle transition section 22 has a higher durometer than its adjacent, previous 21, and next 23, transition sections. That is, the middle transition section is precisely designed to be stiffer than its preceding and following transition sections to enable the catheter to transmit a distally directed pushing force while navigating through tortuous vessels and to thereby enhance longitudinal force transmission from proximal section 10 to distal section 30 without elongation or tension accumulation. In this way, all the force applied at the proximal section 10 is transferred into movement at the distal section 30 despite the tortuosity of the vessels between proximal section 10 and distal section 30.

More in particular, in the embodiment of Fig. 1, the transition section (or fifth transition section) 21A is disposed distal to the proximal section 10 and is less stiff than, or equally stiff to, the proximal section 10; the transition section (or first transition section) 21B is disposed distal to the transition section 21A and is less stiff than, or equally stiff to, the transition section 21A; the transition section (or second transition section) 22 is disposed distal to the transition section 21B and is more stiff than the transition section 21B; the transition section (or third transition section) 23A is disposed distal to the transition section 22 and is less stiff than the transition section 22; the transition section (or fourth transition section) 23B is disposed distal to the transition section 23A and is less stiff than the transition section 23A and more stiff than the distal section 30. This more complex transition structure may enable the catheter to more effectively navigate through tortuous vessels without elongation or tension accumulation.

In some embodiments of Fig. 1, the different durometers, materials and lengths of the different sections of the tubular elongated body can be the following:

**Table 1:**

| SECTION | MATERIAL | DUROMETER | LENGTH |
|---|---|---|---|
| 10 | Extrusion- GRILAMID | 70D | 392 mm |
| 21A | Extrusion- PEBAX | 72D | 450 mm |
| 21B | Extrusion- PEBAX | 55D | 110 mm |
| 22 | Extrusion- PEBAX | 72D | 30 mm |
| 23A | Extrusion- PEBAX | 55D | 50 mm |
| 23B | Extrusion- PEBAX | 40D | 60 mm |
| 30 | Extrusion- Pellethane 80A | 29D | 150 mm |

**Table 2:**

| SECTION | MATERIAL | DUROMETER | LENGTH |
|---|---|---|---|
| 10 | Extrusion- GRILAMID | 70D | 450 mm |
| 21A | Extrusion- PEBAX | 70D | 450 mm |
| 21B | Extrusion- PEBAX | 70D | 110 mm |
| 22 | Extrusion- PEBAX | 72D | 15 mm |
| 23A | Extrusion- PEBAX | 55D | 50 mm |
| 23B | Extrusion- PEBAX | 40D | 50 mm |
| 30 | Extrusion- Pellethane 80A | 29D | 100 mm |

**Table 3:**

| SECTION | MATERIAL | DUROMETER | LENGTH |
|---|---|---|---|
| 10 | Extrusion- GRILAMID | 70-74D | 350-600 mm |
| 21A | Extrusion- PEBAX | 70-72D | 50-600 mm |
| 21B | Extrusion- PEBAX | 55-72D | |
| 22 | Extrusion- PEBAX | 55-72D | 20-200 mm |
| 23A | Extrusion- PEBAX | 55-72D | 30-70 mm |
| 23B | Extrusion- PEBAX | 40-55D | 40-80 mm |
| 30 | Extrusion- Pellethane 80A | 25-30D | 80-170 mm |

In an embodiment, the proximal section 10 has a durometer between 70-74D, and the distal section 30 has a durometer between 25-30D. In the embodiment of Fig. 1, the transition section 21A has a durometer between 70-72D, but lower or equal than the durometer of the proximal section 10; the transition section 21B has a durometer between 55-72D, but lower or equal than the durometer of the transition section 21A; the transition section 22 has a durometer between 55-72D, more particularly between 60-72D, but higher than the durometer of the transition section 21B; the transition section 23A has a durometer between 55-72D, but lower than the durometer of the transition section 22; the transition section 23B has a durometer between 40-55D, but lower than the durometer of the transition section 23A.

In any of the above embodiments, the elongated tubular body can have a length in a range between 900-1500 mm, particularly 1000-1400 mm, for example 1320 mm or 1350 mm.

In an embodiment, the length of the proximal section 10 depends on the overall length of the delivery catheter. In another embodiment, the length of the proximal section 10 ranges between 350-600 mm, for example 450 mm.

In an embodiment, the length of the distal section 30 ranges between 80-170 mm, for example 100 mm or 150 mm.

In an embodiment, the length of the transition section 21B and the length of the transition section 21A depend on the overall length of the delivery catheter. In another embodiment, the length of the transition section 21B and the length of the transition section 21A sum up between 50-600 mm, for example, the length of the transition section 21A and the transition section 21B are 450 mm and 110 mm, respectively.

In an embodiment, the length of the transition section 22 ranges between 20-200 mm, particularly 20-60 mm or 60-200 mm.

In an embodiment, the length of the transition section 23A ranges between 40-80 mm, particularly 40-60 mm or 60-80 mm.

In an embodiment, the length of the transition section 23B ranges between 30-70 mm, particularly 30-50 mm or 50-70 mm.

In one embodiment, the different sections of the delivery catheter have a stiffness as shown in Table 4, wherein the stiffness is measured in terms of peak force [N] as described in Example 1.

**Table 4:**

| SECTION | FORCE | FORCE RANGE | LENGTH | LENGTH RANGE |
|---|---|---|---|---|
| 10 | 1.92 N | 1.46-2.22 N | 392 mm | 350-600 mm |
| 21A | 1.43 N | 1.04-1.75 N | 450 mm | 50-600 mm |
| 21B | 0.66 N | 0.63-1.04 N | 110 mm | |
| 22 | 0.66 N | 0.63-0.69 N | 30 mm | 20-200 mm |
| 23A | 0.63 N | 0.39-0.69 N | 50 mm | 30-70 mm |
| 23B | 0.39 N | 0.31-0.47 N | 60 mm | 40-80 mm |
| 30 | 0.27 N | 0.23-0.31 N | 150 mm | 80-170 mm |

Referring back to Fig. 2, the first transition section 21 is less stiff than the proximal section 10; the second transition section 22 is disposed distal to the first transition section 21 and particularly is more stiff than the first transition section 21; and the third transition section 23 is disposed distal to the second transition section 22 and is less stiff than the second transition section 22.

In some embodiments of Fig. 2, the different durometers, materials and lengths of the different sections of the tubular elongated body can be the following:

**Table 5:**

| SECTION | MATERIAL | DUROMETER | LENGTH |
|---|---|---|---|
| 10 | Extrusion- GRILAMID | 70-74D | 392 mm |
| 21 | Extrusion- PEBAX | 55-72D | 560 mm |
| 22 | Extrusion- PEBAX | 60-72D | 30 mm |
| 23 | Extrusion- PEBAX | 40-55D | 110 nm |
| 30 | Extrusion- Pellethane 80A | 25-30D | 150 mm |

**Table 6:**

| SECTION | MATERIAL | DUROMETER | LENGTH |
|---|---|---|---|
| 10 | Extrusion- GRILAMID | 70-74D | Depending on the overall length of the catheter |
| 21 | Extrusion- PEBAX | 55-74D | 50-600 mm |
| 22 | Extrusion- PEBAX | 55-74D | 15-200 mm |
| 23 | Extrusion- PEBAX | 40-60D | 40-160 nm |
| 30 | Extrusion- Pellethane 80A | 25-55D | 80-170 mm |

**Table 7:**

| SECTION | MATERIAL | DUROMETER | LENGTH |
|---|---|---|---|
| 10 | Extrusion- GRILAMID | 70-74D | Depending on the overall length of the catheter |
| 21 | Extrusion- PEBAX | 55-72D | 110-560 mm |
| 22 | Extrusion- PEBAX | 60-72D | 15-50 mm |
| 23 | Extrusion- PEBAX | 40-60D | 100-150 nm |
| 30 | Extrusion- Pellethane 80A | 25-55D | 100-150 mm |

**Table 8:**

| SECTION | MATERIAL | DUROMETER | LENGTH |
|---|---|---|---|
| 10 | Extrusion- GRILAMID | 70D-74D | 350 - 600 mm |
| 21 | Extrusion- PEBAX | 55D-74D | 50 - 200 mm |
| 22 | Extrusion- PEBAX | 55D-74D | 20 - 200 mm |
| 23 | Extrusion- PEBAX | 40-60D | 40 - 160 mm |
| 30 | Extrusion- Pellethane 80A | 25-30D | 80 - 170 mm |

In an embodiment, the proximal section 10 has a durometer between 70-74D, and the distal section 30 has a durometer between 25-55D. In the embodiment of Fig. 2, the first transition section 21 has a durometer between 55-74D, but lower than the durometer of the proximal section 10; the second transition section 22 has a durometer between 55-74D, but higher than the durometer of the first transition section 21; the third transition section 23 has a durometer between 40-60D, but lower than the durometer of the second transition section 22.

In an embodiment, the length of the proximal section 10 depends on the overall length of the delivery catheter. In another embodiment, the length of the proximal section 10 ranges between 350-600 mm, for example 392 mm.

In an embodiment, the length of the distal section 30 ranges between 80-170 mm, particularly 100-150 mm, for example 100 or 150 mm.

In an embodiment, the length of the first transition section 21 depends on the overall length of the delivery catheter. In another embodiment, the length of the first transition section 21 ranges between 50-600 mm, particularly 110-560 mm, for example 110 or 560 mm.

In an embodiment, the length of the second transition section 22 ranges between 15-200 mm, particularly 15-60 mm or 60-200 mm, for example 15 or 30 mm.

In an embodiment, the length of the third transition section 23 ranges between 40-160 mm, particularly 50-150 mm, for example 50, 60 or 110 mm.

In an embodiment, the elongated tubular body comprises two longitudinal layers, an inner layer made of PTFE and an outer layer made of PEBAX^{®}. In some embodiments, the elongated tubular body further includes an intermediate layer made of PEBAX^{®} disposed between the inner and outer layers.

The elongated tubular body can also comprise a soft-tip zone disposed at the most distal part of the distal section 30. The soft-tip zone particularly has a length of 2 mm and a durometer of 25-30, particularly 29.

The elongated tubular body can also include a reinforcing coil. The reinforcing coil can extend the full length of the elongated tubular body (disposed in the soft-tip zone) or can extend until the distal section 30 only. The reinforcing coil can be disposed between the outer layer and the inner layer or between the outer layer and the intermediate layer.

The reinforcing coil in particular comprises a stainless-steel material. This coil structure underneath the jacket material of the tubular body is designed also to enhance the longitudinal force transmission. The coil structure varies from a small pitch for a higher stiffness at the proximal section 10 to a bigger pitch for a better flexibility at the distal section 30. Also, the coil structure in the second transition section 22 is different to its adjacent transition sections.

In a particular embodiment, the reinforcing coil comprises a 0.010" (0.25 mm) pitch at the proximal section 10, a 0.012" (0.30 mm) pitch at the middle transition section 22 and a 0.015" (0.38 mm) pitch at the distal section 30.

The elongated tubular body can have a diameter on the order of 4 to 6 French. In a particular embodiment the delivery catheter is a 6-French diameter device.

By way of non-limiting example, the elongated tubular body can have an outer diameter between 0.078" and 0.085" (i.e., 2.0-2.18 mm), particularly 0.083" (i.e., 2.11 mm). This diameter is defined by a guiding catheter. The requirement set is that the delivery catheter needs to fit the guiding catheter with a minimum ID of 0.088" (i.e., 2.2 mm). If the diameter of the delivery catheter changes, other parameters (e.g., material/stainless-steel structure) can be readjusted to maintain the flexibility/pushability properties achieved with current design. In some embodiments, the inner diameter of the elongated tubular body is limited by an outer diameter of an aspiration catheter which will be disposed within the delivery catheter. A minimum clearance between them is needed (0.05 mm). In another embodiment, the elongated tubular body can have an inner diameter between 0.064" - 0.073" (i.e., 1.65-1.85 mm), particularly 0.072" (i.e., 1.82 mm). In another embodiment, the thickness of the elongated tubular body is 0.011" (i.e., 0.28 mm).

**Table 9:**

| | Range | | Real (nominal) |
|---|---|---|---|
| Delivery catheter | OD [mm] | 2.0-2.18 | 2.11 |
| | ID [mm] | 1.65-1.85 | 1.82 |

Although the design of the proposed delivery catheter is optimized to access the brain, in particular through the carotid syphon 405, the delivery catheter can be equally used for other applications. In the alternative applications other than the brain the cited diameters can be equal or higher. For example, for the peripheral circulatory system, the diameter can be of 9F (French) = 3 mm. In other applications, for example for accessing renal arteries, gastric arteries, uterine arteries, etc. diameters of 6F = 2mm can be used.

The elongated tubular body can also include a coating, such as a hydrophilic coating. Different hydrophilic materials can be used for example: Polyvinylpyrrolidone, Polyacrylamide or combinations thereof, among others.

After placement of the delivery catheter at a desired location in a vessel of the patient's vasculature, a medical device can be moved through and out of the delivery catheter to that target location. While in the catheter as the catheter moves through the vasculature, the movable medical device can increase the stiffness of the catheter at the location of the medical device. In some embodiments, the movable medical device to be delivered by the delivery catheter has a collapsed delivery configuration, and it self-expands when moved out of the distal end of the delivery catheter into an expanded deployed configuration. While in the delivery configuration, the movable medical device exerts an outward radial force on the delivery catheter, which can add to the stiffness of the catheter at the location of the medical device.

Fig. 3 shows an example of a movable medical device that can be held/accommodated by the proposed delivery catheter. Therefore, in some embodiments of the invention, the system provided by the present invention includes both a delivery catheter and a movable medical device.

The illustrated movable medical device can change its shape from a collapsed position within the elongated tubular body to an extended and expanded position outside of an opening of the distal section 10 to remove a thrombus. In its collapsed delivery configuration, the movable medical device can apply a radially-outward force on the inner surface of the delivery catheter. Thus, the movable medical device can alter the stiffness of the delivery catheter device as it is advanced or retracted through the elongated tubular body. According to the non-claimed method, the movable medical device can be advanced or retracted within the delivery catheter to alter the stiffness of a section of the delivery catheter. For example, the delivery catheter can be advanced through portions of the vasculature with the movable medical device disposed within the distal section of the delivery catheter. When the distal end of the delivery catheter reaches a more tortuous portion of the vasculature, the movable medical device can be retracted a short distance within the delivery catheter to increase the flexibility of the distal portion of the delivery catheter. The movable medical device can be advanced to the distal end of the delivery catheter after the distal end of the delivery catheter has gotten beyond the tortuous vasculature portion.

In particular, the movable medical device illustrated in Fig. 3 includes a segment 100 comprising a mesh 130 having two sets of helicoidal filaments turning respectively in opposite directions and being intertwined. The mesh 130 in an embodiment can follow a diamond-type structure or a regular structure. The density of the mesh 130 defines the elasticity of the segment 100. A mesh angle (β) with regard to a longitudinal direction can be variable.

The helicoidal filaments can be made of a metal (including metal alloys), polymers, a composite including Nitinol or Nitinol/Platinum, among other materials having suitable mechanical properties.

The mesh 130 defines two distinct tubular sections, a first section 200 and a second section 300. The second section 300 provides a reduction of diameter. The second section 300 in some embodiment can comprise two sub sections, a first sub section and a second sub section. The first sub-section (or portion of the second section 300 adjacent to the first section 200) is coneshaped or funnel-shaped. Because of its shape, this sub-section has features enabling it to withstand the blood pressure without collapsing.

The mesh 130 of the first section 200 provides radial forces higher than in the second section 300, such that the first section 200 becomes appositioned against an inner wall of an intracranial blood vessel. The radial forces in first and second end portions of the first section 200 can be higher than in an intermediate portion thereof or the radial forces in the first section 200 can be uniformly distributed along its generatrix.

An end portion of the first section 200 comprises closed loops 230 facilitating the expansion of the segment 100 once it comes out of the delivery catheter. These closed loops 230 act as a spring or fixing point by limiting the movement between the helicoidal filaments and thus increasing the outward radial force.

The distal section 30 and/or the mesh 130 of the movable medical device can include a radiopaque marker. This allows knowing the delivery catheter's position under fluoroscopy and/or knowing when a distal section of the movable medical device is aligned with the distal section 30 of the elongated tubular body.

To deliver segment 100 through a delivery catheter of this invention (such as e.g., the delivery catheters illustrated in Fig. 1 and Fig. 2) to capture a thrombus in an intracranial blood vessel of the patient, the distal section of the delivery catheter can be inserted through an opening into the patient's femoral artery or brachial artery at an insertion site. Segment 100 can be disposed in a collapsed delivery configuration in the distal section 30 of the delivery catheter as the delivery catheter is advanced through the vasculature toward the target location in the intracranial blood vessel. The proximal section 10 of the delivery catheter provides pushability as the delivery catheter and segment 100 are advanced.

When the distal section 30 of the delivery catheter reaches a tortuous portion of a blood vessel, the segment 100 can be withdrawn proximally to decrease the stiffness of the distal section 30. Segment 100 can be advanced distally into the distal section 30 after the distal section 30 is advanced through the tortuous portion of the vessel.

In some vessels, the relative positions of the distal section 30 and the transition sections 20 of the delivery catheter are such that the stiffest transition section of the delivery catheter will be disposed just proximal to a tortuous section of a vessel (e.g., the carotid syphon 405) when the distal section 30 enters that tortuous section. The less stiff section of the delivery catheter proximal to the transition section provides continued flexibility to the transition section of the delivery catheter while the stiffest transition section provides pushability as the distal section of the delivery catheter is advanced through the tortuous section of the vessel.

When the distal section 30 of the delivery catheter reaches the target location in the intracranial vessel, segment 100 can be advanced out of an opening in the distal end of the delivery catheter to self-expand and capture the thrombus. Further details of the movable medical device illustrated in Fig. 3 and its use to capture an intracranial thrombus can be found in US Provisional Application No. 62/760,786, filed November 13, 2018, and International Patent Application WO2020079082A1, filed November 13, 2019.

The embodiments described above are to be understood as a few illustrative examples of the present invention.

Following, different examples of the performance of the proposed delivery catheter device are detailed. The examples and drawings are provided herein for illustrative purposes, and without intending to be limiting to the present invention.

### Example 1: Flexibility testing of commercially available catheter devices in comparison with the ANA system.

The anatomy of the brain presents a very irregular pathways where delivery catheters should navigate to allow the delivery of other coaxial catheters in order to withdrawn thrombus from a target brain location.

Fig. 4A illustrates an example of a 3D in-vitro model of the brain; particularly the Jacobs 3.4 model, where the different segments forming the anatomy of the brain: cervical segment 402, petrous segment 403, cavernous segment 404, carotid syphon 405 and M1 segment 406, are shown. In Fig. 4A, the initial placement 401 of a guiding catheter is also shown.

To obtain a good navigability and pushability of the delivery catheters to reach a target location through the different segments 402-406, the delivery catheters must present different sections with specific stiffness and length, to overcome the tortuous and irregular segments 402-406 of the brain.

The delivery catheters need to be able to navigate until or beyond the carotid syphon 405 no matter where the location of other external coaxial devices (such as a Guide Catheter) are.

The ANA system manufactured by Anaconda Biomed consists of two coaxial catheters called the Delivery Catheter and the Funnel Catheter (according to Fig. 1 and 3, respectively). These two catheters are used together to properly deploy a funnel structure near a clot. The deployment of this structure allows a local flow restriction near the clot area with the aim of proceeding to extract the clot with the aid of a stent retriever and a direct aspiration procedure by the Funnel Catheter.

The purpose of this experimental test was to measure the flexibility in different sections along the whole length of the Delivery Catheter of the ANA system and compare it against other commercially available neurovascular catheters, such as ACE68 (manufactured by Penumbra) and Sofia Plus (manufactured by Terumo). The Delivery Catheter of the ANA system (hereinafter ANA DC) was also evaluated in combination with the Funnel Catheter (hereinafter FC) manufactured by Anaconda Biomed in two different configurations: the ANA DC with the FC deployed (hereinafter as: ANA DC+FC deployed) or the ANA DC with the FC with the distal tip at 5 mm of the distal tip of the DC (hereinafter as: ANA DC+FC 5 mm).

The evaluation of the flexibility was quantified using the 3-point bending (3PB) method in order to provide quantitative insight into the final behavior regarding the navigability, trackability and deliverability of the catheters in a target site (e.g. enable the delivery of the Funnel Catheter).

### Methods

At least 3 units of the following catheters were evaluated; ANA DC, ACE68, and Sofia Plus. ANA system was evaluated in three different configurations: ANA DC alone, ANA DC+FC (with deployed funnel), and ANA DC+FC (with the distal tip of the FC at 5mm of the distal tip of the DC).

The 3PB evaluation was performed in 19 different testing points along the catheter's length. The testing points were defined taking the distal tip of the catheters as a reference (Point 0 cm). The points evaluated in the catheter were: 5, 10, 15, 20, 25, 30, 35, 40, 45, 50, 55, 60, 65, 70, 80, 90, 100, 110, and 120 cm.

The 3PB apparatus (Fig. 5) consists of a means applying and accurately measuring loads and deflections to a specimen mounted in a 3PB bending fixture. The catheters were put over the fixture having two support points with a gauge length of 30mm. The maximum force was recorded when a pusher element with 3.2 mm radius, pushed in the central portion of the specimen by 2mm at a rate of 3 mm/min. This testing method is in accordance with the ASTM F2606-08.

### Results

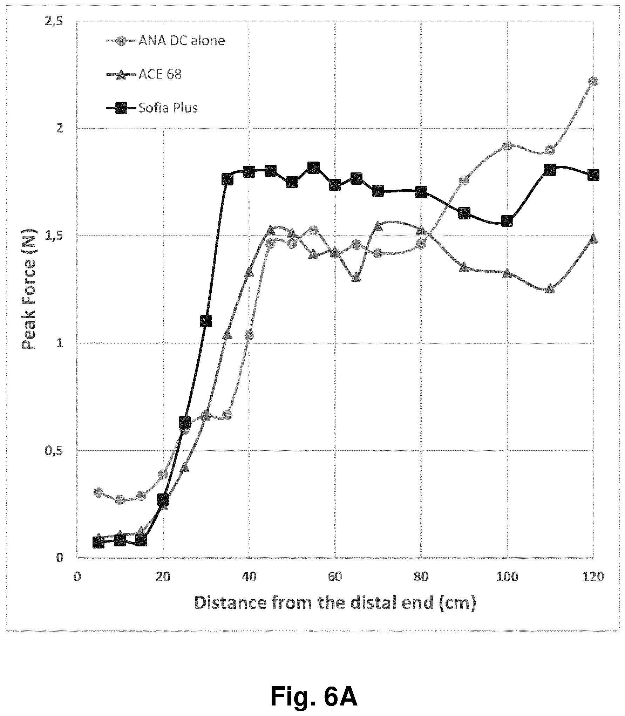

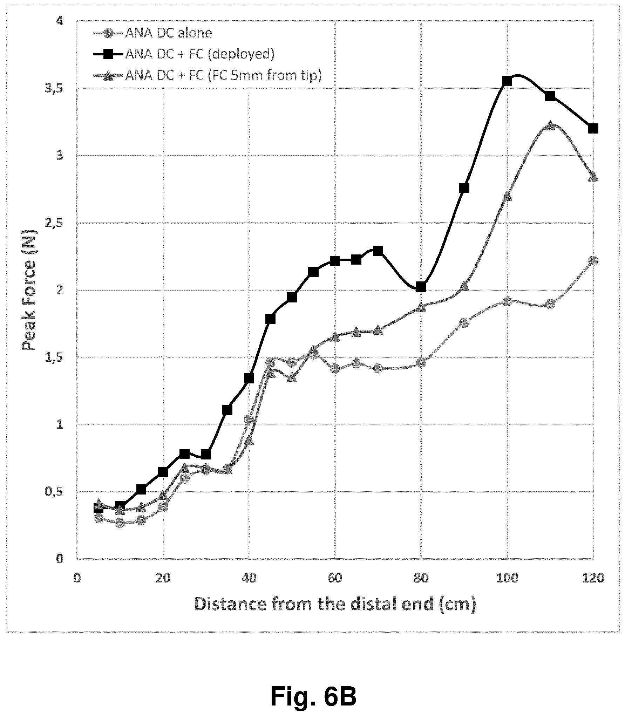

The results, as illustrated in Fig. 6A and Fig. 6B, show the flexibility profile obtained for the different catheters. Mainly, both the most proximal section (2.2-3.2 N) and the most distal section (0.3-0.5 N) of the ANA system in all the studied configurations are stiffer than other commercially available neurovascular catheters, which tend to have similar distal end (<0.1N) and proximal end (1.2-1.8 N) stiffness, making the main decrease from 45 to 20cm from the distal tip. The additional distal stiffness compared with other commercially available devices, avoids the DC to be elongated due to the presence of the funnel in the distal section of the DC, maintaining the integrity of the catheter during the intervention.

Furthermore, with the ANA system (ANA DC alone, ANA DC+FC deployed and ANA DC+FC 5mm) there is a transition near 30cm from the distal tip from a stiffer section to a more flexible section that is intended to transmit the axial movement forces from the proximal to the distal section of the catheter, avoiding, at the same time, possible alterations in the structure of the ANA DC. This transition depends on the structural configuration of the ANA DC, which presents specific stiffness values generating a specific flexibility behavior. On the other hand, the ANA DC+FC (with deployed funnel) and ANA DC+FC (with the distal tip of the FC at 5mm of the distal tip of the DC) configurations resulted slightly stiffer than the ANA DC stand alone.

### Conclusions

3PB tests showed differences in the flexibility profiles between the ANA DC and different commercially available neurovascular catheters.

The ANA system (ANA DC alone, ANA DC+FC deployed and ANA DC+FC 5mm) has a higher stiffness in the distal end and in the proximal end compared to the commercially available neurovascular catheters. The higher stiffness of ANA system in the distal section allows the correct interaction functioning between the ANA DC and the FC, avoiding the elongation of the devices, which it seems not possible with the other commercially available neurovascular catheters (ACE68 and Sofia Plus). Moreover, the transition section present at near 30 cm from the distal tip is intended to correctly transmit axial forces from the proximal end to the distal end of the ANA DC and this improves the pushability and navigability of the ANA DC, which in conjunction with the FC can slightly change its stiffness to adjust the pushability depending on the segment 401-406 of the brain which is passing through.

In conclusion, all these aforementioned features allow the ANA system to reach the brain target site passing through the tortuous vascularity without altering its integrity (i.e. elongation) due to the improved pushability and navigability generated by the interaction of the devices since the Funnel Catheter is configured to alter the stiffness of a portion of the ANA DC as it moves through that portion of the ANA DC.

This study shows that commercially available delivery catheters seem not suitable to deliver and navigate (without alteration of their integrity, i.e. elongation) with the Funnel Catheter manufactured by Anaconda Biomed to a brain target location, e.g. beyond the carotid syphon.

### Example 2: Tensile testing of commercially available catheter devices in comparison with the Delivery Catheter of the ANA system.

The purpose of this experimental test was to measure the tensile force in the distal section of the Delivery Catheter of the ANA system (described in Example 1, hereinafter ANA DC) and compare it against other commercially available neurovascular catheters, such as ACE68 (manufactured by Penumbra, hereinafter ACE) and React 71 (manufactured by Medtronic, hereinafter React).

The evaluation of the tensile force curve in the distal section was quantified in order to provide quantitative insight regarding the capability of the catheters to withstand the forces to move the Funnel Catheter (FC) of the ANA system described in Example 1 inside its structure and also to determine the desired flexibility for the final design of the ANA DC in order to avoid elongation during neurovascular interventions.

### Methods

At least 3 units (n>=3) of the following catheters were evaluated; ANA DC, ACE and React. The tensile testing evaluation was performed at 10 cm position from the distal tip of the catheters. The tensile testing apparatus consists of a means applying and accurately measuring axial loads to a specimen gripped by both sides with a gauge length distance of 10mm. The maximum force was recorded when the gripping elements separate from each other at a rate of 200 mm/min. This testing method is in accordance with the ISO 10555-1.

### Results

Table 10 shows the force measured at 25% of elongation to have a correlation with the elastic behavior of the devices. In this case, as higher the force, the catheter has higher axial resistance.

**Table 10.**

| **Force [N] at 25% Elongation (10 cm from distal tip)** | | | | |
|---|---|---|---|---|
| **Device** | **Average** | **Minimum** | **Maximum** | **Std. Dev.** |
| ANA DC (n=3) | 5.30 | 5.21 | 5.38 | 0.09 |
| ACE (n=4) | 2.44 | 2.12 | 2.73 | 0.29 |
| React (n=4) | 1.81 | 0.86 | 2.32 | 0.65 |

ANA DC obtained the higher value of axial resistance, so it is the most suitable catheter to carry another medical device inside avoiding at the same time the elongation of the catheter during the intervention.

### Conclusions

Tensile tests showed differences between the axial resistance for ANA Delivery Catheter and different commercially available neurovascular catheters. The lower values of forces in React and ACE makes these catheters structures not compatible with the Funnel Catheter because they would suffer from elongation during the intervention due to the movement of the Funnel Catheter. In conclusion, commercially available catheters are not suitable to carry the Funnel Catheter of the ANA system inside.

Finally, Example 1 and 2 showed good results for the ANA Delivery Catheter to maintain its integrity (i.e. without significant elongation) during interventions and is the best candidate to accommodate a Funnel Catheter inside its structure.

Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter. Throughout the description and claims the word "comprise" and its variations such as "comprising" are not intended to exclude other technical features, additives, components, or steps. Additional objects, advantages and features of the invention will become apparent to those skilled in the art upon examination of the description or may be learned by practice of the invention.

The scope of the present invention is defined in the following set of claims.

## Claims

1. A delivery catheter device for accessing intracranial vasculature and for delivering a movable medical device beyond a carotid syphon, comprising:
an elongated tubular body having a lumen, a proximal section (10), a distal section (30) and at least three longitudinally arranged transition sections (20) disposed between the proximal section (10) and the distal section (30), the distal section (30) being the most flexible section of the elongated tubular body, and the proximal section (10) being the stiffest section of the elongated tubular body;
wherein a first transition section (21, 21B) of the longitudinally arranged transition sections (20) has a stiffness less than a stiffness of the proximal section (10), a second transition section (22) of the longitudinally arranged transition sections (20) is disposed distal to the first transition section (21, 21B) and has a stiffness greater than the stiffness of the first transition section (21, 21B), and a third transition section (23, 23A) of the longitudinally arranged transition sections (20) is disposed distal to the second transition section (22) and has a stiffness less than the stiffness of the second transition section (22),
so that the proximal section (10) and the transition sections (20) of the delivery catheter are configured to enable the distal section (30) to be advanced beyond the carotid syphon when a distally directed force on the proximal section (10) is applied.

2. The delivery catheter of claim 1, wherein the longitudinally arranged transition sections (20) further comprise:
a fourth transition section (23B) disposed distal to the third transition section (23A) and having a stiffness less than the stiffness of the third transition section (23A) and greater than the stiffness of the distal section (30), and
a fifth transition section (21A) disposed proximal to the first transition section (21B) and having a stiffness less than the stiffness of the proximal section (10) and greater than the stiffness of the first transition section (21B).

3. The delivery catheter device of claim 1 or 2, wherein:
the proximal section (10) has a durometer in a range comprised between 70-74D, the distal section (30) has a durometer in a range comprised between 25-55D, the first transition section (21, 21B) has a durometer in the range between 55-74D, the second transition section (22) has a durometer in a range comprised between 55-74D, and the third transition section (23, 23A) has a durometer in a range comprised between 40-60D; and
the distal section (30) has a length in a range comprised between 80-170 mm, the first transition section (21, 21B) has a length in a range comprised between 50-600 mm, the second transition section (22) has a length in a range comprised between 15-200 mm, and the third transition section (23, 23A) has a length in a range comprised between 40-160 mm.

4. The delivery catheter device of any one of claims 1 or 2, wherein the distal section (30) has a durometer up to 35D.

5. The delivery catheter device of any one of claims 1 or 2, wherein the distal section (30) and the third transition section (23, 23A) have a durometer up to 55D.

6. The delivery catheter device of any one of claims 1-5, wherein the proximal section (10) is made of a polyamide 12 material, the longitudinally arranged transition sections (20) are made of a polyether block amide material and the distal section (30) is made of a thermoplastic polyurethane material.

7. The delivery catheter device of any one of claims 2-6, wherein the proximal section (10) has a durometer of 70D, the distal section (30) has a durometer of 29D, and the five transition sections (21A, 21B, 22, 23A, 23B) have durometers of 72D, 55D, 72D, 55D, and 40D, respectively, starting from the transition section adjacent to the proximal section (10).

8. The delivery catheter device of any of claims 1-6, wherein the proximal section (10) has a durometer in a range between 70-74D, the distal section (30) has a durometer in a range between 25-30D, the third transition section (23) next to the distal section (30) has a durometer in a range between 40-60D, the second transition section (22) has a durometer in a range between 55-74D, and the first transition section (21) next to the proximal section (10) has a durometer in a range between 55-74D.

9. The delivery catheter device of any one of the previous claims, wherein the elongated tubular body has an outer diameter in a range between 2.0-2.18 mm and an inner diameter in a range between 1.65-1.85 mm.

10. The delivery catheter device of any one of the previous claims, wherein the elongated tubular body further comprises a reinforcing coil, wherein a section of the reinforcing coil in the transition sections (20) has a stiffness that is different than a stiffness of a section of the reinforcing coil in the proximal section (10) and a stiffness of a section of the reinforcing coil in the distal section (30).

11. The delivery catheter of any one of the previous claims, wherein the movable medical device is disposed within the distal section (30), the proximal section (10) and the transition sections (20) of the delivery catheter being configured to enable the distal section (30) to be advanced beyond the carotid syphon when a distally directed force on the proximal section (10) is applied.

12. The delivery catheter device of any one of the previous claims, wherein it is adapted to hold the movable medical device within the elongated tubular body and to place said movable medical device in an intracranial blood vessel to remove a thrombus, such that the stiffness of a portion of the delivery catheter device is altered by the movable medical device as it moves through that portion of the elongated tubular body.

13. The delivery catheter device of any one of the previous claims, further comprising a radiopaque marker at the distal section (30) of the elongated tubular body to allow viewing a position of the delivery catheter device within the intracranial blood vessel under fluoroscopy.

14. A system for accessing the intracranial vasculature and for delivering a movable medical device beyond a carotid syphon, comprising:
a movable medical device;
a delivery catheter device comprising an elongated tubular body having a lumen, a proximal section (10), a distal section (30) and at least three longitudinally arranged transition sections (20) disposed between the proximal section (10) and the distal section (30), wherein the distal section (30) is the most flexible section of the elongated tubular body, wherein the proximal section (10) is the stiffest section of the elongated tubular body;
the delivery catheter is adapted to hold said movable medical device within the elongated tubular body and to place it in an intracranial blood vessel to remove a thrombus;
the movable medical device is adapted to change its shape from a collapsed position within the elongated tubular body to an extended and expanded position at an opening of the distal section (30) to remove said thrombus;
wherein a first transition section (21, 21B) of the longitudinally arranged transition sections (20) has a stiffness less than a stiffness of the proximal section (10), a second transition section (22) of the longitudinally arranged transition sections (20) is disposed distal to the first transition section (21, 21B) and has a stiffness greater than the stiffness of the first transition section (21, 21B), and a third transition section (23, 23A) of the longitudinally arranged transition sections (20) is disposed distal to the second transition section (22) and has a stiffness less than the stiffness of the second transition section (22), and
so that the proximal section (10) and the transition sections (20) of the delivery catheter are configured to enable the distal section (30) to be advanced beyond the carotid syphon when a distally directed force on the proximal section (10), when the movable medical device is disposed within the distal section (30), is applied.

15. The system of claim 14, wherein the longitudinally arranged transition sections (20) further comprise:
a fourth transition section (23B) disposed distal to the third transition section (23A) and having a stiffness less than the stiffness of the third transition section (23A) and greater than the stiffness of the distal section (30), and
a fifth transition section (21A) disposed proximal to the first transition section (21B) and having a stiffness less than the stiffness of the proximal section (10) and greater than the stiffness of the first transition section (21B).

## Patentansprüche

1. Abgabekathetervorrichtung zum Zugriff auf das intrakranielle Gefäßsystem und zum Abgeben einer beweglichen medizinischen Vorrichtung über ein Karotissiphon hinaus, Folgendes umfassend:
einen langgestreckten rohrförmigen Körper mit einem Lumen, einem proximalen Abschnitt (10), einem distalen Abschnitt (30) und mindestens drei in Längsrichtung angeordneten Übergangsabschnitten (20), die zwischen dem proximalen Abschnitt (10) und dem distalen Abschnitt (30) angeordnet sind, wobei der distale Abschnitt (30) der flexibelste Abschnitt des langgestreckten rohrförmigen Körpers ist und der proximale Abschnitt (10) der steifste Abschnitt des langgestreckten rohrförmigen Körpers ist;
wobei ein erster Übergangsabschnitt (21, 21B) der in Längsrichtung angeordneten Übergangsabschnitte (20) eine Steifigkeit aufweist, die geringer als die Steifigkeit des proximalen Abschnitts (10) ist, ein zweiter Übergangsabschnitt (22) der in Längsrichtung angeordneten Übergangsabschnitte (20) distal zu dem ersten Übergangsabschnitt (21, 21B) angeordnet ist und eine Steifigkeit aufweist, die größer als die Steifigkeit des ersten Übergangsabschnitts (21, 21B) ist, und ein dritter Übergangsabschnitt (23, 23A) der in Längsrichtung angeordneten Übergangsabschnitte (20) distal zu dem zweiten Übergangsabschnitt (22) angeordnet ist und eine Steifigkeit aufweist, die geringer als die Steifigkeit des zweiten Übergangsabschnitts (22) ist,
so dass der proximale Abschnitt (10) und die Übergangsabschnitte (20) des Abgabekatheters so ausgebildet sind, dass der distale Abschnitt (30) über das Karotissiphon hinaus vorgeschoben werden kann, wenn eine distal gerichtete Kraft auf den proximalen Abschnitt (10) angewendet wird.

2. Abgabekatheter nach Anspruch 1, wobei die in Längsrichtung angeordneten Übergangsabschnitte (20) ferner Folgendes umfassen:
einen vierten Übergangsabschnitt (23B), der distal zu dem dritten Übergangsabschnitt (23A) angeordnet ist und eine Steifigkeit aufweist, die geringer als die Steifigkeit des dritten Übergangsabschnitts (23A) und größer als die Steifigkeit des distalen Abschnitts (30) ist, und
einen fünften Übergangsabschnitt (21A), der proximal zu dem ersten Übergangsabschnitt (21B) angeordnet ist und eine Steifigkeit aufweist, die geringer als die Steifigkeit des proximalen Abschnitts (10) und größer als die Steifigkeit des ersten Übergangsabschnitts (21B) ist.

3. Abgabekathetervorrichtung nach Anspruch 1 oder 2, wobei:
der proximale Abschnitt (10) einen Durometer in einem zwischen 70-74 D umfassenden Bereich aufweist, der distale Abschnitt (30) einen Durometer in einem zwischen 25-55 D umfassenden Bereich aufweist, der erste Übergangsabschnitt (21, 21B) einen Durometer in dem zwischen 55-74 D umfassenden Bereich aufweist, der zweite Übergangsabschnitt (22) einen Durometer in einem zwischen 55-74 D umfassenden Bereich aufweist und der dritte Übergangsabschnitt (23, 23A) einen Durometer in einem zwischen 40-60 D umfassenden Bereich aufweist; und
der distale Abschnitt (30) eine Länge in einem zwischen 80-170 mm umfassenden Bereich aufweist, der erste Übergangsabschnitt (21, 21B) eine Länge in einem zwischen 50-600 mm umfassenden Bereich aufweist, der zweite Übergangsabschnitt (22) eine Länge in einem zwischen 15-200 mm umfassenden Bereich aufweist, und der dritte Übergangsabschnitt (23, 23A) eine Länge in einem zwischen 40-160 mm umfassenden Bereich aufweist.

4. Abgabekathetervorrichtung nach einem der Ansprüche 1 oder 2, wobei der distale Abschnitt (30) einen Durometer von bis zu 35 D aufweist.

5. Abgabekathetervorrichtung nach einem der Ansprüche 1 oder 2, wobei der distale Abschnitt (30) und der dritte Übergangsabschnitt (23, 23A) einen Durometer von bis zu 55 D aufweisen.

6. Abgabekathetervorrichtung nach einem der Ansprüche 1-5, wobei der proximale Abschnitt (10) aus einem Polyamid-12-Material, die in Längsrichtung angeordneten Übergangsabschnitte (20) aus einem Polyetherblockamid-Material und der distale Abschnitt (30) aus einem thermoplastischen Polyurethan-Material hergestellt sind.

7. Abgabekathetervorrichtung nach einem der Ansprüche 2-6, wobei der proximale Abschnitt (10) einen Durometer von 70 D aufweist, der distale Abschnitt (30) einen Durometer von 29 D aufweist und die fünf Übergangsabschnitte (21A, 21B, 22, 23A, 23B) Durometer von jeweils 72 D, 55 D, 72 D, 55 D und 40 D aufweisen, beginnend mit dem an den proximalen Abschnitt (10) angrenzenden Übergangsabschnitt.

8. Abgabekathetervorrichtung nach einem der Ansprüche 1-6, wobei der proximale Abschnitt (10) einen Durometer in einem Bereich zwischen 70-74 D aufweist, der distale Abschnitt (30) einen Durometer in einem Bereich zwischen 25-30 D aufweist, der dritte Übergangsabschnitt (23) neben dem distalen Abschnitt (30) einen Durometer in einem Bereich zwischen 40-60 D aufweist, der zweite Übergangsabschnitt (22) einen Durometer in einem Bereich zwischen 55-74 D aufweist, und der erste Übergangsabschnitt (21) neben dem proximalen Abschnitt (10) einen Durometer in einem Bereich zwischen 55-74 D aufweist.

9. Abgabekathetervorrichtung nach einem der vorhergehenden Ansprüche, wobei der langgestreckte rohrförmige Körper einen Außendurchmesser in einem Bereich zwischen 2,0-2,18 mm und einen Innendurchmesser in einem Bereich zwischen 1,65-1,85 mm aufweist.

10. Abgabekathetervorrichtung nach einem der vorhergehenden Ansprüche, wobei der langgestreckte rohrförmige Körper ferner eine Verstärkungsspirale aufweist, wobei ein Abschnitt der Verstärkungsspirale in den Übergangsabschnitten (20) eine Steifigkeit aufweist, die sich von einer Steifigkeit eines Abschnitts der Verstärkungsspirale im proximalen Abschnitt (10) und einer Steifigkeit eines Abschnitts der Verstärkungsspirale im distalen Abschnitt (30) unterscheidet.

11. Abgabekatheter nach einem der vorhergehenden Ansprüche, wobei die bewegliche medizinische Vorrichtung innerhalb des distalen Abschnitts (30) angeordnet ist, wobei der proximale Abschnitt (10) und die Übergangsabschnitte (20) des Abgabekatheters so ausgebildet sind, dass der distale Abschnitt (30) über das Karotissiphon hinaus vorgeschoben werden kann, wenn eine distal gerichtete Kraft auf den proximalen Abschnitt (10) angewendet wird.

12. Abgabekathetervorrichtung nach einem der vorhergehenden Ansprüche, wobei sie dazu ausgelegt ist, die bewegliche medizinische Vorrichtung innerhalb des langgestreckten rohrförmigen Körpers zu halten und die bewegliche medizinische Vorrichtung in einem intrakraniellen Blutgefäß zu platzieren, um einen Thrombus zu entfernen, so dass die Steifigkeit eines Teils der Abgabekathetervorrichtung durch die bewegliche medizinische Vorrichtung verändert wird, während sie sich durch diesen Teil des langgestreckten rohrförmigen Körpers bewegt.

13. Abgabekathetervorrichtung nach einem der vorhergehenden Ansprüche, die ferner einen röntgendichten Marker am distalen Abschnitt (30) des langgestreckten rohrförmigen Körpers umfasst, um die Position der Abgabekathetervorrichtung innerhalb des intrakraniellen Blutgefäßes unter Fluoroskopie sichtbar zu machen.

14. System zum Zugriff auf das intrakranielle Gefäßsystem und zum Abgeben einer beweglichen medizinischen Vorrichtung über ein Karotissiphon hinaus, Folgendes umfassend:
eine bewegliche medizinische Vorrichtung;
eine Abgabekathetervorrichtung, umfassend einen langgestreckten rohrförmigen Körper mit einem Lumen, einem proximalen Abschnitt (10), einem distalen Abschnitt (30) und mindestens drei in Längsrichtung angeordneten Übergangsabschnitten (20), die zwischen dem proximalen Abschnitt (10) und dem distalen Abschnitt (30) angeordnet sind, wobei der distale Abschnitt (30) der flexibelste Abschnitt des langgestreckten rohrförmigen Körpers ist, wobei der proximale Abschnitt (10) der steifste Abschnitt des langgestreckten rohrförmigen Körpers ist;
wobei der Abgabekatheter so ausgelegt ist, dass er die bewegliche medizinische Vorrichtung innerhalb des langgestreckten rohrförmigen Körpers hält und sie in einem intrakraniellen Blutgefäß platziert, um einen Thrombus zu entfernen;
wobei die bewegliche medizinische Vorrichtung so ausgelegt ist, dass sie ihre Form aus einer kollabierten Position innerhalb des langgestreckten rohrförmigen Körpers in eine ausgefahrene und expandierte Position an einer Öffnung des distalen Abschnitts (30) verändert, um den Thrombus zu entfernen;
wobei ein erster Übergangsabschnitt (21, 21B) der in Längsrichtung angeordneten Übergangsabschnitte (20) eine Steifigkeit aufweist, die geringer als die Steifigkeit des proximalen Abschnitts (10) ist, ein zweiter Übergangsabschnitt (22) der in Längsrichtung angeordneten Übergangsabschnitte (20) distal zu dem ersten Übergangsabschnitt (21, 21B) angeordnet ist und eine Steifigkeit aufweist, die größer als die Steifigkeit des ersten Übergangsabschnitts (21, 21B) ist, und ein dritter Übergangsabschnitt (23, 23A) der in Längsrichtung angeordneten Übergangsabschnitte (20) distal zu dem zweiten Übergangsabschnitt (22) angeordnet ist und eine Steifigkeit aufweist, die geringer als die Steifigkeit des zweiten Übergangsabschnitts (22) ist, und
so dass der proximale Abschnitt (10) und die Übergangsabschnitte (20) des Abgabekatheters so ausgebildet sind, dass der distale Abschnitt (30) über das Karotissiphon hinaus vorgeschoben werden kann, wenn eine distal gerichtete Kraft auf den proximalen Abschnitt (10) angewendet wird, wenn die bewegliche medizinische Vorrichtung innerhalb des distalen Abschnitts (30) angeordnet ist.

15. System nach Anspruch 14, wobei die in Längsrichtung angeordneten Übergangsabschnitte (20) ferner Folgendes umfassen:
einen vierten Übergangsabschnitt (23B), der distal zu dem dritten Übergangsabschnitt (23A) angeordnet ist und eine Steifigkeit aufweist, die geringer als die Steifigkeit des dritten Übergangsabschnitts (23A) und größer als die Steifigkeit des distalen Abschnitts (30) ist, und
einen fünften Übergangsabschnitt (21A), der proximal zu dem ersten Übergangsabschnitt (21B) angeordnet ist und eine Steifigkeit aufweist, die geringer als die Steifigkeit des proximalen Abschnitts (10) und größer als die Steifigkeit des ersten Übergangsabschnitts (21B) ist.

## Revendications

1. Un dispositif pour un cathéter de mise en place pour accéder au système vasculaire intracrânien et pour mettre en place un dispositif médical mobile au-delà d'un siphon carotidien, comportant.
un corps tubulaire allongé ayant un lumen, une section proximale (10), une section distale (30) et, au moins, trois sections de transition (20) aménagées longitudinalement disposées entre la section proximale (10) et la section distale (30), la section distale (30) étant la section la plus flexible du corps tubulaire allongé, et la section proximale (10) étant la section la plus raide du corps tubulaires allongé ;
où une première section de transition (21, 21B) des sections de transition (20) aménagée longitudinalement a une raideur inférieure à une raideur de la section proximale (10), une deuxième section de transition (22) des sections de transition (20) aménagées longitudinalement est disposée distale de la première section de transition (21, 21B) et a une raideur supérieure à la raideur de la première section de transition (21, 21B), et une troisième section de transition (23, 23A) des sections de transition aménagées longitudinalement (20) est disposée distale de la deuxième section de transition (22) et a une raideur inférieure à la deuxième section de transition (22),
de sorte que la section proximale (10) et les sections de transition (20) du cathéter de mise en place sont configurées pour permettre que la section distale (30) avance au-delà du siphon carotidien lorsqu'une force distalement dirigée sur la section proximale (10) est appliquée.

2. Le cathéter de mise en place de la revendication 1, où les sections de transition aménagées longitudinalement (20) comportent en outre :
- une quatrième section de transition (23B) disposée distale de la troisième section de transition (23A) et ayant une raideur inférieure à la raideur de la troisième section de transition (23A) et supérieure à la raideur de la section distale (30), et
une cinquième section de transition (21A) disposée proximale de la première section de transition (21B) et ayant une raideur inférieure à la raideur de la section proximale (10) et supérieure à la raideur de la première section de transition (21B).

3. Le cathéter de mise en place de la revendication 1 ou 2, où :
la section proximale (10) a un duromètre d'une gamme comprise entre 70 et 74D, la section distale (30) a un duromètre d'une gamme comprise entre 25 et 55D, la première section de transition (21, 21B) a un duromètre d'une gamme entre 55 et 74D, la deuxième section de transition (22) a un duromètre d'une gamme comprise entre 55 et 74D, et la troisième section de transition (23, 23A) a un duromètre d'une gamme comprise entre 40 et 60D ; et
la section distale (30) a une longueur d'une gamme comprise entre 80 et 170 mm, la première section de transition (21, 21B) a une longueur d'une gamme entre 50 et 600mm, la deuxième section de transition (22) a une longueur d'une gamme comprise entre 15 et 200 mm et la troisième section de transition (23, 23A) a une longueur d'une gamme comprise entre 40 et 160 mm.

4. Le cathéter de mise en place d'une quelconque des revendications 1 ou 2, où la section distale (30) a un duromètre de jusqu'à 35D.

5. Le cathéter de mise en place d'une quelconque des revendications 1 ou 2, où la section distale (30) et la troisième section de transition (23, 23A) ont un duromètre de jusqu'à 55D.

6. Le cathéter de mise en place d'une quelconque des revendications 1-5, où la section proximale (10) est faite en un matériau de polyamide 12, les sections de transition aménagées longitudinalement (20) sont faites en un matériau d'amide de bloc de polyester et la section distale (30) est faite en un matériau de polyuréthane thermoplastique.

7. Le cathéter de mise en place d'une quelconque des revendications 2-6, où la section proximale (10) a un duromètre de 70D, la section distale (30) a un duromètre de 29D, et les cinq sections de transition (21A, 21B, 22, 23A, 23B) ont des duromètres de 72D, 55D, 72D, 55D et 40D, respectivement, en commençant depuis la section de transition adjacente à la section proximale (10).

8. Le cathéter de mise en place d'une quelconque des revendications 1-6, où la section proximale (10) a un duromètre d'une gamme entre 70 et 74D, la section distale (30) a un duromètre d'une gamme entre 25 et 30D, la troisième section de transition (23) à côté de la section distale (30) a un duromètre d'une gamme entre 40 et 60D, la deuxième section de transition (22) a un duromètre d'une gamme ente 55 et 74D, et la première section de transition (21) , à côté de la section proximale (10) a un duromètre d'une gamme entre 55 et 74D.

9. Le cathéter de mise en place d'une quelconque des revendications précédentes, où le corps tubulaire allongé a un diamètre extérieur d'une gamme entre 2,0 et 2,18 mm et un diamètre intérieur d'une gamme entre 1,65 et 1,85 mm.

10. Le cathéter de mise en place d'une quelconque des revendications précédentes, où le corps tubulaire allongé comprend en outre une bobine de renforcement, où une section de la bobine de renforcement dans les sections de transition (20) a une raideur qui est différente d'une raideur d'une section de la bobine de renforcement dans la section proximale (10) et une raideur d'une section de la bobine de renforcement dans la section distale (30).

11. Le cathéter de mise en place d'une quelconque des revendications précédentes, où le dispositif médical mobile est disposé à l'intérieur de la section distale (30), la section proximale (10) et les sections de transition (20) du cathéter de mise en place étant configurés pour permettre que la section distale (30) avance au-delà du siphon carotidien lorsqu'une force dirigée distalement sur la section proximale (10) est appliquée.

12. Le cathéter de mise en place d'une quelconque des revendications précédentes, où il est apte pour tenir le dispositif médical mobile à l'intérieur du corps tubulaire allongé et de placer ce dispositif médical mobile dans un vaisseau sanguin intracrânien pour retirer un thrombus, de sorte que la raideur d'une portion du dispositif de cathéter de mise en place est altérée par le dispositif médical mobile au fur et à mesure qu'il se déplace à travers cette portion du corps tubulaire allongé.

13. Le cathéter de mise en place d'une quelconque des revendications précédentes, comprenant en outre un marqueur radio opaque à la section distale (30) du corps tubulaire allongé pour permettre voir une position du dispositif de cathéter de mise en place à l'intérieur du vaisseau sanguin intracrânien sous fluoroscopie.

14. Un système pour avoir accès au système vasculaire intracrânien et pour mettre en place un dispositif médical mobile au-delà du siphon carotidien, comprenant :
un dispositif médical mobile ;
un dispositif de cathéter de mise en place comprenant un corps tubulaire allongé ayant un lumen , une section proximale (10), une section distale (30) et au moins trois sections de transition (20) aménagées longitudinalement, disposées entre la section proximale (10) et la section distale (30), où la section distale (30) est la section la plus flexible du corps tubulaire allongé, où la section proximale (10) est la section la plus raide du corps tubulaire allongé ;
le cathéter de mise en place est apte pour tenir ce dispositif médical mobile à l'intérieur du corps tubulaire allongé et le placer dans un vaisseau sanguin intracrânien ou pour enlever un thrombus ;
le dispositif médical mobile est apte pour changer sa forme d'une position repliée à l'intérieur du corps tubulaire allongé à une position étendue et élargie à une ouverture de la section distale (30) pour enlever ce thrombus ;
où une première section de transition (21, 21B) des sections de transition aménagées longitudinalement (20) a une raideur inférieure à une raideur de la section proximale (10), une deuxième section de transition (22) des sections de transition aménagées longitudinalement (20) est disposée distale de la première section de transition (21, 21B) et a une raideur supérieure à la raideur de la première section de transition (21, 21B), et une troisième section de transition (23, 23A) des sections de transition aménagées longitudinalement (20) est disposée distale de la deuxième section de transition (22) et a une raideur inférieure à la raideur de la deuxième section de transition (22) ; et
de sorte que la section proximale (10) et les sections de transition (20) du cathéter de mise en place sont configurées pour permettre que la section distale (30) avance au-delà du siphon carotidien lorsqu'une force distalement dirigée sur la section proximale (10), lorsque le dispositif médical mobile est disposé à l'intérieur de la section distale (30), est appliquée.

15. Le système de la revendication 14, où les sections de transition longitudinalement aménagées (20) comprennent, en outre :
une quatrième section de transition (23B) disposée distale de la troisième section de transition (23A) et ayant une raideur inférieure à la raideur de la troisième section de transition (23A) et supérieure à la raideur de la section distale (30), et
une cinquième section de transition (21A) disposée proximale à la première section de transition (21B) et ayant une raideur inférieure à la raideur de la section proximale (10) et supérieure à la raideur de la première section de transition (21B).
